# EUROPÄISCHE PATENTSCHRIFT

(11) **EP 3 061 473 B1**
(45) Veröffentlichungstag und Bekanntmachung des Hinweises auf die Patenterteilung: **15.05.2019**
(21) Anmeldenummer: 16155912.5
(22) Anmeldetag: 16.02.2016
(51) Int. Cl.: A61M 1/10

(54) **SCHLAUCHROLLENPUMPE MIT WINKELVARIABLEN ANDRUCKROLLEN**
PERISTALTIC PUMP WITH VARIABLE ANGLE PRESSURE ROLLERS
POMPE A GALETS TUBULAIRE DOTEE DE ROULEAUX DE PRESSION A ANGLE VARIABLE

(30) Priorität: 25.02.2015 DE 102015102659
(43) Veröffentlichungstag der Anmeldung: 31.08.2016
(73) Patentinhaber: B. Braun Avitum AG, 34212 Melsungen (DE)
(72) Erfinder: SCHÄFER, Oliver, 36286 Neuenstein (DE); RITTER, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- DE-A1- 2 452 771
- DE-A1- 2 543 797
- JP-A- S57 122 187

## Beschreibung

Die Erfindung betrifft ein Verfahren zum Fördern von Fluid, insbesondere Blut, in einer Vorrichtung zur extrakorporalen Blutbehandlung, insbesondere in einer Dialysemaschine, wobei Fluid mittels einer Peristaltikpumpe von einer Niederdruckseite zu einer Hochdruckseite gefördert wird und eine elastisch verformbare und zwischen der Niederdruckseite und der Hochdruckseite angeordnete Fluidleitung zwischen einer Stützfläche und einem gegenüber dieser rotierenden Rotor mit zumindest zwei Quetschelementen verformt wird, insbesondere zusammengequetscht wird. Sie betrifft des Weiteren eine Dialysemaschine mit einer Fluid von einer Niederdruckseite zu einer Hochdruckseite fördernden Peristaltikpumpe, wobei die Peristaltikpumpe eine elastisch verformbare Fluidleitung zwischen der Niederdruckseite und der Hochdruckseite, eine die Fluidleitung stützende Stützfläche und einen Rotor aufweist, wobei der Rotor zumindest zwei jeweils ein die Fluidleitung zwischen sich und der Stützfläche verformende Quetschelemente, insbesondere zusammenquetschende Quetschelemente, aufweist. Außerdem betrifft die Erfindung eine Steuereinrichtung für eine Dialysemaschine.

Derartige Verfahren und Dialysemaschinen sind aus dem Stand der Technik bekannt. Die Peristaltikpumpe eines solchen Systems hat die Aufgabe, ein definiertes Volumen eines Mediums, wie zum Beispiel Blut oder Dialysefluid, durch Verformen und Abklemmen der elastisch verformbaren Fluidleitung zu fördern. Eine Peristaltikpumpe zur Förderung von Blut fördert in der Regel von einer negativen Druckseite (Niederdruckseite) auf eine positive Druckseite (Hochdruckseite). Bekannte Systeme bei Medizingeräten zur extrakorporalen Blutbehandlung bestehen in der Regel aus einem Rotor, einem Pumpengehäuse und einer zwischen diesen angeordneten Schlauchleitung, und fördern ein definiertes Volumen unter starrem, d.h. konstantem Druck von der Niederdruckseite auf die Hochdruckseite. Dabei stehen im Falle einer Peristaltikpumpe mit zwei Quetschelementen diese im Stand der Technik häufig in einer relativen Position von 180° starr bzw. in einem festen Winkel von 180° zueinander. Es ist aus dem Stand der Technik, beispielsweise aus der DE 24 52 771 A1 und der JP S57 122187 A, auch bekannt, dass die Quetschelemente während der Rotation des Rotors zum Bewirken einer Vorkompression zueinander winkelpositioniert werden. Einen weiteren Stand der Technik stellt die DE 25 43 797 A1 dar.

Es ist ein leider häufig bei bekannten Dialysemaschinen und Verfahren auftretender Nachteil, dass es während eines Pumpvorganges im geförderten Fluidvolumen zu unerwünschter Pulsation auf der Hochdruckseite kommen kann. Dieser nachteilige Effekt kommt daher, dass niederdruckseitig ein Volumenabschnitt der elastischen Fluidleitung abgeklemmt wird und das darin eingeschlossene Fluidvolumen durch Rotation des Rotors und dadurch bedingte Verlagerung der Quetschstelle der Fluidleitung in Richtung der Hochdruckseite gefördert wird. Bei Abklemmen des Fördervolumenabschnitts steht das darin eingeschlossene Volumen unter niederdruckseitigem Druck. Es wird unter diesem Druck auf die Hochdruckseite gefördert, wo hingegen Hochdruck herrscht. Wird im Rahmen des Fördervorgangs der Fördervolumenabschnitt zur Hochdruckseite geöffnet, kommt es aufgrund der Druckdifferenz zwischen der Hochdruckseite und dem Fördervolumenabschnitt zu einer Fluidströmung von der Hochdruckseite in den Fördervolumenabschnitt, bis Druckausgleich vorliegt. Folge ist ein kurzzeitiges Einbrechen des hochdruckseitigen Drucks und es kommt zu Pulsation auf der Hochdruckseite.

Ein weiterer nachteiliger Effekt bei bekannten Verfahren und Dialysemaschinen ist, dass im Falle einer Förderung von Blut als Fluid während des vorstehend beschriebenen Druckausgleichs Blut durch die sich öffnende Engstelle zwischen dem Fördervolumenabschnitt und der Hochdruckseite gequetscht wird. Dabei kommt es in der Regel zur teilweisen Zerstörung von Blutzellen, allgemein als Hämolyse bezeichnet.

Ausgehend von dem vorstehend beschriebenen Stand der Technik liegt der vorliegenden Erfindung die Aufgabe zugrunde, die zuvor angeführten Nachteile zu beseitigen, insbesondere den vorstehend beschriebenen negativen Pulsationseffekt zu minimieren.

Diese Aufgabe wird gelöst durch ein Verfahren zum Fördern von Fluid in einer Vorrichtung zur extrakorporalen Blutbehandlung nach Anspruch 1, eine Dialysemaschine mit einer Fluid von einer Niederdruckseite zu einer Hochdruckseite fördernden Peristaltikpumpe nach Anspruch 7 und einer Steuereinrichtung für eine Dialysemaschine nach Anspruch 10. Vorteilhafte Ausführungsformen der Erfindung sind in den Unteransprüchen beansprucht.

Die Erfindung betrifft ein Verfahren zum Fördern von Fluid in einer Vorrichtung zur extrakorporalen Blutbehandlung, wobei Fluid mittels einer Peristaltikpumpe von einer Niederdruckseite zu einer Hochdruckseite gefördert wird, wobei eine elastisch verformbare und zwischen der Niederdruckseite und der Hochdruckseite angeordnete Fluidleitung zwischen einer Stützfläche und einem gegenüber dieser rotierenden Rotor mit zumindest zwei Quetschelementen (bzw. Andruckrollen) verformt wird, bei dem die Quetschelemente während der Rotation des Rotors zum Bewirken einer Vorkompression relativ zueinander winkelpositioniert werden. Außerdem betrifft die Erfindungeine Dialysemaschine mit einer Fluid von einer Niederdruckseite zu einer Hochdruckseite fördernden Peristaltikpumpe, wobei die Peristaltikpumpe eine elastisch verformbare Fluidleitung zwischen der Niederdruckseite und der Hochdruckseite, eine die Fluidleitung stützende Stützfläche und einen Rotor aufweist, wobei der Rotor zumindest zwei jeweils ein die Fluidleitung zwischen sich und der Lauffläche verformende Quetschelemente aufweist, bei der die Quetschelemente in Rotationsrichtung relativ zueinander winkelpositionierbar ausgebildet sind.

Nach der Erfindung erfolgt eine Vorkompression eines von der Niederdruckseite auf die Hochdruckseite geförderten Fluidvolumenabschnitts während der Förderung. Der Rotor und die die elastische Fluidleitung stützende Stützfläche sind derart ausgebildet und aufeinander abgestimmt, dass zwischen ihnen eine Förderstrecke ausgebildet ist. Im Bereich der Förderstrecke wird die Fluidleitung zwischen der Stützfläche und einem Quetschelement quer zu ihrem Querschnitt verformt, insbesondere zusammengequetscht oder fluiddicht zusammengequetscht. Ein vorlaufendes Quetschelement läuft erst aus der Förderstrecke aus, wenn ein nachlaufendes Quetschelement in die Förderstrecke eingelaufen ist. Anders ausgedrückt ist der Winkel zwischen Förderstreckeneinlauf und Förderstreckenauslauf größer als der Winkel zwischen einem vorlaufenden Quetschelement und einem nachlaufenden Quetschelement. Es gibt daher nach der Erfindung stets einen Zeitraum bzw. eine Förderstreckenabschnitt, bei dem ein zwischen vorlaufenden Quetschelement und nachlaufenden Quetschelement gefördertes Fluidvolumen zwischen diesen eingeschlossen ist. Dieser Zeitraum bzw. dieser Förderstreckenabschnitt wird nach der Erfindung genutzt, um das geförderte Fluidvolumen derart vorzukomprimieren, dass Druckschwankungen auf der Hochdruckseite minimiert und vorzugsweise eliminiert werden. Anders ausgedrückt wird durch geometrische Abhängigkeiten das abgenommene Volumen von der negativen Druckseite bis zur Abgabe des Volumens auf der positiven Druckseite komprimiert und somit eine Pulsierung minimiert.

Nach der Erfindung weist der Rotor zumindest zwei Quetschelemente auf. Dies ist die geringstmögliche Anzahl an Quetschelementen, die zur Ausbildung einer definierten, insbesondere einer abgedichteten Förderstrecke erforderlich ist. Es liegt im Bereich der Erfindung, wenn der Rotor mehr als zwei Quetschelemente aufweist, insbesondere drei oder vier. Die Winkelpositionen der Quetschelemente zueinander, also die zwischen benachbarten Quetschelementen befindlichen Winkel, betragen außerhalb des Bereichs der Vorkompression vorzugsweise im Falle von zwei Quetschelementen 180°, im Falle von drei Quetschelementen 120° und Falle von vier Quetschelementen 90°. Die Länge der Förderstrecke der Fluidleitung ist in allen Fällen größer.

Die Vorkompression erfolgt, indem der Winkelabstand benachbarter Quetschelemente, also der Winkel zwischen einem vorlaufenden Quetschelement und dem diesen nachlaufenden Quetschelement, verringert wird, solange sich beide Quetschelemente im Förderstreckenabschnitt befinden, dieser also durch beide Quetschelemente zu beiden Seiten geschlossen ist. Eine Verringerung des Abstandes der betreffenden Quetschelemente führt zu einer Verringerung des Volumens des Förderstreckenabschnitts. Da aufgrund dessen Abdichtung mittels der Quetschelemente kein Fluid entweichen kann, bis das vorlaufende Quetschelement aus dem Förderstreckenabschnitt ausläuft, ist ein Druckanstieg die Folge. Die Abstandsverringerung der betreffenden Quetschelemente ist derart ausgewählt, dass eine Druckdifferenz zwischen dem Förderstreckenabschnitt und der Hochdruckseite verringert, vorzugsweise ausgeglichen wird.

Die Quetschelemente können unmittelbar am Rotor ausgebildet sein, insbesondere einstückig mit dem Rotor. Sie können alternativ auf Rotorarmen angeordnet sein. Diese sind vorzugsweise gegenüber dem Rotor in umfänglicher Richtung verschwenkbar ausgebildet, so dass über ein Verschwenken in Umfangsrichtung die Vorkompression erzielt werden kann. Die Quetschelemente können insbesondere als Quetschrollen oder Andruckrollen, die in vorteilhafter Weise materialschonend an der Fluidleitung abrollen, oder Gleitschuhe, die sich gleitend über die Fluidleitung bewegen, ausgebildet sein.

Mit der Erfindung können insbesondere die folgenden Vorteile erzielt werden:
- Reduktion bzw. Vermeidung von blutschädigender Hämolyse, da ein Rückströmen von Fluid vom Hochdruckbereich in die Förderstrecke aufgrund von Druckdifferenz verringert oder vermieden wird,
- Druckausgleich zwischen Niederdruckseite und Hochdruckseite aufgrund der vorstehend beschriebenen Vorkompression,
- Verringerung oder sogar Vermeidung von Pulsation während des Pumpvorgangs.

Bei einer Ausführungsform wird das im Förderstreckenabschnitt befindliche Fluidvolumen komprimiert, indem das nachlaufende Quetschelement in Richtung des vorlaufenden Quetschelements vorgestellt wird. Infolgedessen wird das zwischen den beiden Quetschelementen eingeschlossene Volumen verringert und das darin vorliegende Fluid vorkomprimiert. Anders ausgedrückt rotiert das nachlaufende Quetschelement nach Einschließen des zu fördernden Fluidvolumens für einen bestimmten Zeitraum schneller als das vorlaufende Quetschelement um die Rotorachse, bis die erwünschte Vorkompression erreicht ist.

Bei einer anderen alternativen Ausführungsform kann vorgesehen sein, dass das vorlaufende Quetschelement in Richtung des nachlaufenden Quetschelements nachgestellt wird. Infolgedessen wird ebenfalls das zwischen den beiden Quetschelementen eingeschlossene Volumen verringert und das darin vorliegende Fluid vorkomprimiert. Anders ausgedrückt rotiert das vorlaufende Quetschelement nach Einschließen des zu fördernden Fluidvolumens für einen bestimmten Zeitraum langsamer als das nachlaufende Quetschelement um die Rotorachse oder bleibt (für kurze Zeit) stehen, bis die erwünschte Vorkommpression erreicht ist. Eine Kombination der beiden vorgenannten Ausführungsformen ist außerdem im Bereich der Erfindung.

Nach einer Ausführungsform der Erfindung werden der niederdruckseitige Druck und der hochdruckseitige Druck erfasst und es wird eine Druckdifferenz gebildet. Die Winkelpositionierung der Quetschelemente zueinander kann dann abhängig von dieser Druckdifferenz erfolgen. Diese Druckerfassung ist in vorteilhafter Weise einfach, da die Drücke auf der Hoch- sowie auf der Niederdruckseite infolge guter Zugänglichkeit einfach zu messen sind.

Bei einer anderen Ausführungsform der Erfindung werden der hochdruckseitige Druck und der Druck im geförderten Fluidvolumen erfasst und es wird eine Druckdifferenz daraus gebildet. Die Winkelpositionierung der Quetschelemente zueinander erfolgt abhängig von dieser Druckdifferenz. Bei dieser Ausführungsform ist besonders vorteilhaft, dass aufgrund der Erfassung des Drucks im Förderstreckenabschnitt eine Vorkompression und damit eine Druckkompensierung besonders genau erfolgen können.

In einer weiteren Ausführungsform kann der hochdruckseitige Druckverlauf erfasst werden und die Winkelpositionierung der Quetschelemente zueinander in Abhängigkeit vom hochdruckseitigen Druckverlauf erfolgen. Hier ist von Vorteil, dass eine Druckerfassung nur an einer Stelle im System zu erfolgen hat, wodurch das System besonders einfach und robust ausgebildet werden kann.

Zur Druckerfassung in einer der vorbeschriebenen Weisen weist die Dialysemaschine des Weiteren ein Druckmessmittel zur Feststellung des einlassseitigen Drucks und/oder ein Druckmessmittel zur Feststellung des ausgangsseitigen Drucks und/oder ein Druckmessmittel zur Feststellung des Drucks im Pumpsegment, d.h. in der in der Fluidleitung ausgebildeten Förderstrecke, auf.

Bei einer Form der Erfindung kann das aus der Förderstrecke auslaufende Quetschelement nach Auslaufen aus dem Förderstreckenabschnitt relativ zu dem vorlaufenden Quetschelement in eine neutrale Position überführt werden. Dies ermöglicht eine besonders leichte Steuerung bzw. Einstellung der Vorkompression.

Es ist von besonderem Vorteil, wenn die Quetschelemente mit einem Kurvenstellglied, insbesondere einer Kurven- oder Nockenscheibe oder einer Kurven- oder Nockenwelle, zusammenwirken, wobei die Winkelposition der Quetschelemente zueinander mittels des Kurvenstellgliedes einstellbar ist. Über eine Variation der Kurvengeometrie kann die Vorkompression sehr einfach und reproduzierbar eingestellt werden. Alternativ kann jedes Quetschelement jeweils mittels einer Antriebseinheit, insbesondere mittels eines Schrittmotors angetrieben sein. Das bewirkt den Vorteil, dass eine Einstellung oder eine Variation der Vorkompression besonders einfach zu steuern ist.

Anders ausgedrückt bezieht sich die Erfindung auf einen druckausgleichenden Rotor, welcher Bestandteil einer peristaltisch arbeitenden (Schlauch-)Rollenpumpe, insbesondere Schlauchpumpe für die Medizintechnik ist, die ihren bestimmungsgemäßen Einsatz in der extrakorporalen Blutbehandlung findet. Dieser Rotor ermöglicht zusammen mit den elastischen Materialeigenschaften des Pumpensegmentes eines Überleitsystems, welches schlaufenförmig gegen eine zylindrische Lauffläche des Pumpengehäuses eingelegt wird, eine Pumpfunktion, die eine Blutförderung zu einem Dialysator sicherstellt. Man kann auch sagen, dass die vorliegende Erfindung die ihr zugrundeliegende Aufgabe löst, indem der Rotor innerhalb einer Umdrehung (360°) mehrfach die relative Position der Quetschelemente zueinander verändert. Dies bedeutet, dass zum Beispiel bei einem Rotor mit zwei Quetschelementen diese bei dem Aufnehmen des Volumens auf der negativen Druckseite eine relative Position von 180° zueinander haben. Nachdem das Volumen durch das zweite Quetschelement eingeschlossen ist, verändert sich die relative Position der Quetschelemente zueinander auf kleiner 180°. Dadurch erhöht sich der Druck des im Förderabschnitt der Fluidleitung eingeschlossenen Volumens. Idealerweise wird die Erhöhung des Drucks so ausgelegt, dass sich der Druck im eingeschlossenen Segment möglichst dem Druck auf der Pumpenausgangsseite annähert. Nach Abgabe des Volumens auf der positiven Druckseite kann sich die relative Position der Quetschelemente zueinander wieder auf 180° ändern. Diese zyklischen Positionsänderungen der Quetschelemente können beispielsweise durch eine geometrisch definierte Kontur, zum Beispiel eine Kurvenscheibe oder eine Nockengeometrie, auf einen zum Beispiel zweigeteilten und drehbar gelagerten Rotor übertragen werden.

Es liegt insbesondere im Bereich der Erfindung, dass der Rotor zwei jeweils ein Quetschelement tragende Arme aufweist. Diese können insbesondere einzeln, beispielsweise durch einen Schrittmotor, angetrieben sein bzw. werden, so dass die Stellung zueinander frei gesteuert werden kann. Wird der ein- und ausgangsseitige Druck gemessen, wie es heute auch üblich ist, so ist es vorteilhaft, den Voreilwinkel der zweiten Rolle druckdifferenzabhängig einstellbar zu machen. Ziel ist es, die Pulsation zu minimieren oder gar auszulöschen. Des Weiteren kann mittels eines Drucksensors auf der Ausgangsseite die Pulsation ermittelt werden und der Voreilwinkel auf minimale Pulsation eingeregelt werden. In diesem Arbeitspunkt tritt dann auch die geringste Hämolyse auf.

Ein weiterer Aspekt der Erfindung betrifft eine Steuereinrichtung für eine Dialysemaschine, welche eine Peristaltikpumpe aufweist, die ein Fluid von einer Niederdruckseite zu einer Hochdruckseite fördert, wobei die Peristaltikpumpe eine elastisch verformbare Fluidleitung zwischen der Niederdruckseite und der Hochdruckseite, eine die Fluidleitung stützende Stützfläche und einen Rotor aufweist, wobei der Rotor zumindest zwei jeweils ein die Fluidleitung zwischen sich und der Stützfläche verformende Quetschelemente aufweist, wobei jedes Quetschelement jeweils mittels einer Antriebseinheit, insbesondere mittels eines Schrittmotors, angetrieben ist, und wobei die Dialysemaschine ein Druckmessmittel zur Feststellung des einlassseitigen Drucks und/oder ein Druckmessmittel zur Feststellung des ausgangsseitigen Drucks und/oder ein Druckmessmittel zur Feststellung des Drucks in der Fluidleitung aufweist. Erfindungsgemäß steuert die Steuereinrichtung zum Bewirken einer Vorkompression zumindest eine Antriebseinheit derart, dass der Relativwinkel in Rotationsrichtung zwischen den Quetschelementen auf der Grundlage des festgestellten einlassseitigen Drucks und/oder des festgestellten ausgangsseitigen Drucks und/oder des festgestellten Drucks in der Fluidleitung verändert wird, insbesondere verkleinert wird.

Die Erfindung wird im Folgenden anhand beispielhafter, nicht einschränkender und in den angehängten Figuren gezeigter Ausführungsformen näher erläutert. Dabei zeigt:
Figur 1 eine schematische Darstellung eines Ausschnitts einer Vorrichtung zur extrakorporalen Blutbehandlung in einer beispielhaften Ausführungsform,
Figur 2 eine beispielhafte schematische Darstellung einer Regelstrecke im Sinne der Erfindung,
Figur 3 eine schematische Aufsicht auf eine Peristaltikpumpe nach der Erfindung in einem ersten Zustand zu einem ersten Zeitpunkt des Betriebs,
Figur 4 eine schematische Aufsicht auf die Peristaltikpumpe der Figur 3 in einem auf den ersten Zustand folgenden zweiten Zustand zu einem zweiten Zeitpunkt des Betriebs,
Figur 5 eine schematische Aufsicht auf die Peristaltikpumpe der Figur 3 in einem auf den zweiten Zustand folgenden dritten Zustand zu einem dritten Zeitpunkt des Betriebs und
Figur 6 eine schematische Aufsicht auf die Peristaltikpumpe der Figur 3 in einem auf den dritten Zustand folgenden vierten Zustand zu einem vierten Zeitpunkt des Betriebs.

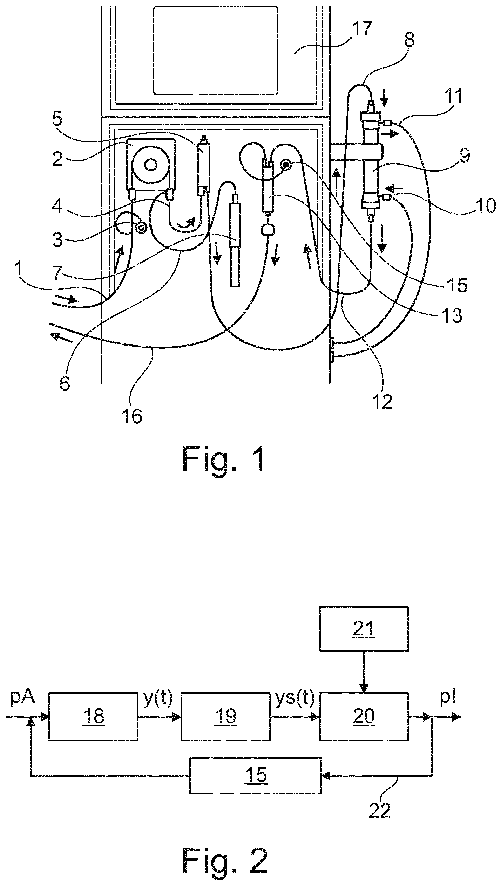

Figur 1 zeigt beispielhaft einen Ausschnitt einer Vorrichtung zur extrakorporalen Blutbehandlung nach der Erfindung. Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige Druck gemessen wird. Hochdruckseitig der Peristaltikpumpe 2 führt eine Hochdruckblutleitung 4 zu einem arteriellen Luftfänger 5. Unmittelbar am Ausgang der Peristaltikpumpe 2 kann mittels einer Zuleitung 6 und einer Pumpe 7 Zusatzstoff in das im System befindlichen Blut gegeben werden, z.B. Heparin zur Blutverdünnung.

Vom arteriellen Luftfänger 5 führt eine Leitung 8 unter Hochdruck stehendes aber noch unbehandeltes Blut zu einem Dialysator 9. Diesem wird eingangsseitig Dialysierflüssigkeit über eine Dialysierflüssigkeitszuleitung 10 zugeführt. Im Dialysator 9 wird Blut in bekannter Weise mittels der Dialysierflüssigkeit behandelt, z.B. gereinigt. Verbrauchte Dialysierflüssigkeit wird über eine Dialysierflüssigkeitsableitung 11 vom Dialysator 9 entfernt und einer nicht dargestellten Entsorgung oder Aufbereitung zugeführt. Behandeltes Blut wird mittels einer Blutableitung 12 vom Dialysator 9 zu einem venösen Luftfänger 13 geleitet und mittels dessen Luft abgeschieden wird. Am venösen Luftfänger 13 ist ein venöser Druckaufnehmer 15 vorgesehen, mit dem der venöse Druck, also der hochdruckseitige Druck, erfasst wird. Vom venösen Luftfänger 13 wird behandeltes Blut über eine venöse Blutleitung 16 zurück zum Patienten geleitet. Dargestellt in Figur 1 ist auch eine Einheit 17 zur Überwachung und Steuerung der Vorrichtung.

Die Überwachungseinheit 17 dient unter anderem der Durchführung des in Figur 2 dargestellten Regelkreises. Die zu regelnde Größe pA ist der Solldruck am Ausgang der Blutpumpe 2, also der Druck in der Hochdruckblutleitung 4. Mittels eines Reglers 18 wird eine Stellgröße y(t) eines Schrittmotors 19 beeinflusst und als ys(t) in eine Regelstrecke 20 eingebracht. Auf diese wirken Störgrößen 21, z.B. in Form sich ändernder Temperatur oder einer langsam fortschreitenden Alterung der Leitungen der Vorrichtung, ein. Aus der Regelstrecke heraus kommt die Ist-Größe pl, also der Istdruck am Ausgang der Blutpumpe 2. Dieser wird mittels des Druckaufnehmers 15 erfasst und über eine Rückführung 22 wieder in die Regelstrecke eingebracht. Es sei angemerkt, dass der Druckaufnehmer 15 oder ein zusätzlicher Druckaufnehmer anders als in Figur 1 beispielhaft gezeigt unmittelbar hinter der Peristaltikpumpe 2 vorgesehen sein kann.

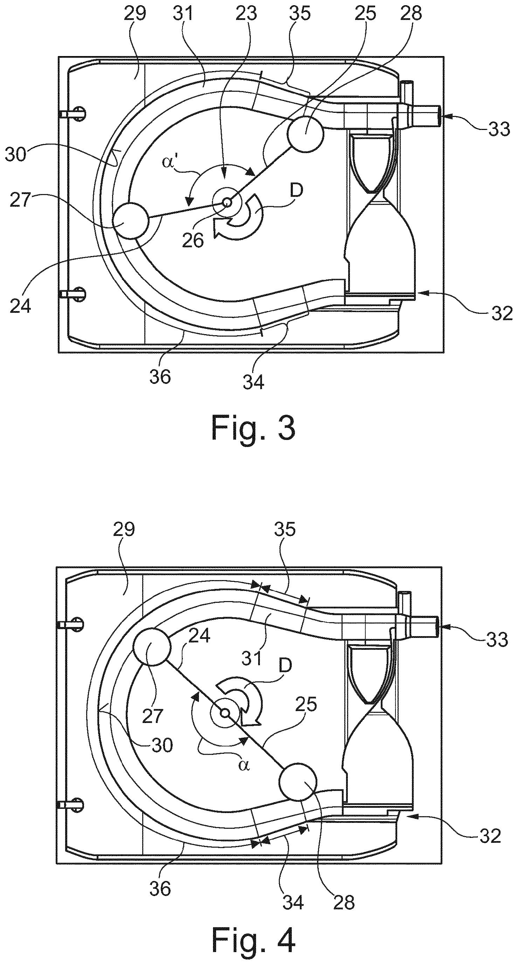

Die Figuren 3 bis 6 zeigen die Peristaltikpumpe 2 mit der arteriellen Blutleitung 1 und der Hochdruckblutleitung 4 in einem Ausschnitt zu verschiedenen Zeitpunkten während des Verfahrens nach der Erfindung. Die Peristaltikpumpe 2 weist einen Rotor 23 mit einem ersten Rotorarm 24 und einem zweiten Rotorarm 25 auf. Die Rotorarme 24, 25 rotieren um eine gemeinsame Rotorachse 26. Der erste Rotorarm 24 trägt auf seiner der Rotorachse 26 abgewandten Seite ein erstes Quetschelement 27 in Form einer ersten Quetschrolle 27. Der zweite Rotorarm 25 trägt auf seiner der Rotorachse 26 abgewandten Seite ein zweites Quetschelement 28 in Form einer zweiten Quetschrolle 28. Die Peristaltikpumpe 2 weist des Weiteren ein Blutpumpengehäuse 29 auf, das in bekannter Weise eine Stützfläche 30 ausbildet. Im Blutpumpengehäuse 29 ist eine Fluidleitung 31 angeordnet, derart, dass sie zwischen der Stützfläche 30 und den Quetschelementen 27, 28 deformiert wird. Sie wird derart insbesondere elastisch deformiert, dass ihr Querschnitt teilweise zusammengequetscht, also verengt wird, insbesondere vollständig zusammengequetscht, also im Wesentlichen fluiddicht geschlossen wird.

Die Fluidleitung 31 ist an der Seite ihres Einlaufs 32 mit der arteriellen Blutleitung 1 und an der Seite ihres Auslaufs 33 mit der Hochdruckblutleitung 4 verbunden. Die Fluidleitung 31 ist in einem Teilbereich zwischen einem Einlaufabschnitt 34 und einem Auslaufabschnitt 35 in Form eines Teilkreises angeordnet. Der Einlaufabschnitt 34 reicht von dem Bereich der Fluidleitung 31, in dem die Quetschelemente 27, 28 bei rotierendem Rotor 23 mit dieser in Kontakt gelangen, bis zu dem Bereich der Fluidleitung 31, in dem die Verformung des Querschnitts der Fluidleitung 31 durch die Quetschelemente 27, 28 abgeschlossen ist. Umgekehrt reicht der Auslaufabschnitt 35 von dem Bereich der Fluidleitung 31, in dem die Verformung des Querschnitts der Fluidleitung 31 durch die Quetschelemente 27, 28 noch vollständig vorliegt, bis zu dem Bereich der Fluidleitung 31, in dem die Quetschelemente 27, 28 bei rotierendem Rotor 23 den Kontakt zur Fluidleitung verlieren. Zwischen dem Einlaufabschnitt 34 und dem Auslaufabschnitt 35 bildet die Fluidleitung 31 einen Förderstreckenabschnitt 36 aus. In Figur 3 sind der Förderstreckenabschnitt 36, der Einlaufabschnitt 34 und der Auslaufabschnitt 35 hinsichtlich ihrer Erstreckung über den jeweiligen Winkelbereich um die Rotorachse 26 gekennzeichnet.

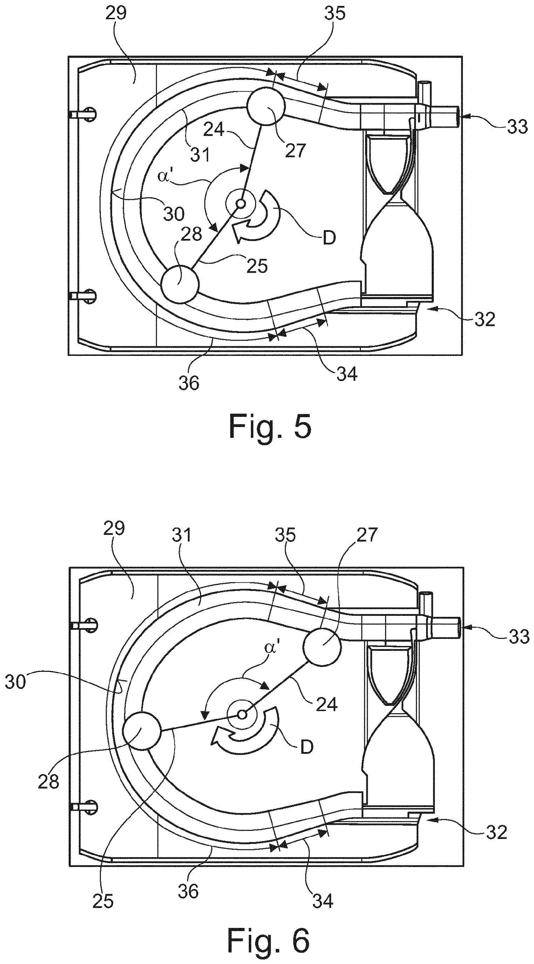

Figur 3 zeigt die Peristaltikpumpe 2 kurz vor dem Ausstoßen des komprimierten Fördervolumens. Dieser Zustand wird im Nachfolgenden noch genauer zusammen mit dem in Figur 6 gezeigten Zustand erläutert.

Figur 4 zeigt die Peristaltikpumpe 2 zu einem Betriebszeitpunkt, zu dem das Quetschelement 28 gerade in den Einlaufabschnitt 34 einläuft und beginnt, die Fluidleitung 31 zu komprimieren und deren Querschnitt zu verengen. Das Quetschelement 27 befindet sich im Bereich des Förderstreckenabschnitts 36 und quetscht die Fluidleitung 31 derart zusammen, dass ihr Querschnitt an der Stelle des Eingriffs des Quetschelements 27 im Wesentlichen vollständig geschlossen ist. In dieser Konfiguration bildet das Quetschelement 27 das vorlaufende Quetschelement aus, während das Quetschelement 28 das nachlaufende Quetschelement ist. Der in Figur 4 eingezeichnete Winkel α zwischen den Rotorarmen 24, 25 (und damit zwischen den Quetschelementen 27, 28) beträgt in diesem Zustand 180° und die Quetschelemente 27, 28 befinden sich in der sogenannten neutralen Position zueinander.

Ein etwas späterer Zeitpunkt ist in Figur 5 gezeigt. Der Rotor 23 hat sich in der angezeigten Drehrichtung D weiter gedreht. Das vorlaufende Quetschelement 27 befindet sich zu dem dargestellten Zeitpunkt genau am Beginn des Auslaufabschnitts 35 der Förderstrecke 36 und verschließt weiterhin den Querschnitt der Fluidleitung 31 an der Stelle seines Eingriffs fluiddicht. Das nachlaufende Quetschelement 28 ist weiter in den Förderstreckenabschnitt 36 eingedrungen und verschließt nun ebenfalls den Querschnitt der Fluidleitung 31 an der Stelle des Eingriffs fluiddicht. Zwischen dem vorlaufenden Quetschelement 27 und dem nachlaufenden Quetschelement 28 ist ein beidseitig durch den Eingriff der Quetschelemente 27, 28 abgedichtetes Fluidvolumen oder Fluidfördervolumen gebildet.

Aus einem Vergleich der Figur 4 mit Figur 5 ist gut zu erkennen, dass während der Rotation von dem in Figur 4 gezeigten Zustand in den in Figur 5 gezeigten Zustand der Winkel α zwischen den beiden Quetschelementen auf den Betrag a' verkleinert wurde (a'<a oder α'+Δα=α). Dies wird erreicht, indem entweder das nachlaufende Quetschelement 28 über einen bestimmten Winkelbereich oder Zeitraum schneller um die Rotorachse 26 rotiert als das vorlaufende Quetschelement 27 und/oder indem das vorlaufende Quetschelement 27 über einen bestimmten Winkelbereich oder Zeitraum langsamer rotiert als das nachlaufende Quetschelement 28 und/oder indem das vorlaufende Quetschelement 27 über einen bestimmten (kurzen) Zeitraum stehen bleibt. Die Winkeländerung Δα bewirkt eine Verringerung des zwischen den beiden Quetschelementen 27, 28 eingeschlossenen Förderfluidvolumens. Da aufgrund der fluiddichten Abdichtung der beiden Enden des Förderfluidvolumens mittels der Quetschelemente 27, 28 kein Fluid aus dem Fördervolumen gelangen kann, kommt es zu der beabsichtigten Vorkomprimierung. Die Größe der Winkeländerung Δα ist derart ausgewählt, dass der Druck in der Förderstrecke im Wesentlichen dem Druck auf der Hochdruckseite entspricht oder diesem zumindest bestmöglich angenähert ist.

Im Verlauf der weiteren Rotation des Rotors 23 in die in Figur 6 gezeigte Stellung, in der sich das vorlaufende Quetschelement 27 am Ende des Auslaufabschnitts 35 befindet, wird die Fluidleitung 31 im Bereich des Eingriffs des Quetschelements 27 geöffnet, so dass Fluid aus dem Fördervolumen in die hochdruckseitige Fluidleitung 4 strömen kann. Da im Fördervolumen im Wesentlichen der gleiche Druck wie in der hochdruckseitigen Blutleitung 4 vorliegt, kommt es beim Öffnen des Fördervolumenabschnitts durch Lösen des Quetschelements 27 von der Fluidleitung 31 zu keiner oder nur zu einer geringen Druckschwankung. Der in Figur 6 gezeigte Zustand entspricht dem in Figur 3 gezeigten Zustand mit der Ausnahme, dass der Rotor 23 um 180° gedreht wurde und in Figur 3 das Quetschelement 28 (anstelle des Quetschelements 27) aus dem Auslaufabschnitt 35 ausläuft. Es sei darauf hingewiesen, dass im Verlauf der weiteren Rotation von dem in Figur 6 gezeigten Zustand sich der Winkel zwischen dem Quetschelement 27 und dem Quetschelement 28 wieder vom Betrag a' auf den Betrag α vergrößert, im dargestellten Beispiel auf 180° und das Quetschelement 27 in einer derartigen Konstellation wieder in den Einlaufabschnitt 34 einläuft und mit der Fluidleitung 31 in Kontakt gelangt. Die vorstehend zwischen den Quetschelementen 27 und 28 ablaufende Vorkompression wiederholt sich in gleicher Weise zwischen den Quetschelementen 28 und 27.

## Patentansprüche

1. Verfahren zum Fördern von Fluid in einer Vorrichtung zur extrakorporalen Blutbehandlung, wobei
Fluid mittels einer Peristaltikpumpe (2) von einer Niederdruckseite zu einer Hochdruckseite gefördert wird, und
eine elastisch verformbare und zwischen der Niederdruckseite und der Hochdruckseite angeordnete Fluidleitung (31) zwischen einer Stützfläche (33) und einem gegenüber dieser rotierenden Rotor (23) mit zumindest zwei Quetschelementen (27, 28) verformt wird, wobei
die Quetschelemente (27, 28) während der Rotation des Rotors (23) zum Bewirken einer Vorkompression relativ zueinander winkelpositioniert werden,
**dadurch gekennzeichnet, dass**
ein niederdruckseitiger Druck und ein hochdruckseitiger Druck erfasst werden, eine Druckdifferenz gebildet wird und eine Winkelpositionierung der Quetschelemente (27, 28) zueinander abhängig von dieser Druckdifferenz erfolgt.

2. Verfahren nach Anspruch 1, wobei zwischen zwei benachbarten Quetschelementen (27, 28) ein zu förderndes Fluidvolumen eingeschlossen wird und nachfolgend eine Volumenkompression des eingeschlossenen Fluidvolumens erfolgt, indem der Winkelabstand α dieser Quetschelemente (27, 28) zueinander verringert wird.

3. Verfahren nach Anspruch 1 oder 2, wobei das nachlaufende Quetschelement (27, 28) in Richtung des vorlaufenden Quetschelements (27, 28) vorgestellt wird.

4. Verfahren nach einem der vorstehenden Ansprüche 1 bis 3, wobei der hochdruckseitige Druck und der Druck im geförderten Fluidvolumen erfasst werden, eine Druckdifferenz gebildet wird und die Winkelpositionierung der Quetschelemente (27, 28) zueinander abhängig von dieser Druckdifferenz erfolgt.

5. Verfahren nach einem der vorstehenden Ansprüche 1 bis 3, wobei der hochdruckseitige Druckverlauf erfasst wird und die Winkelpositionierung der Quetschelemente (27, 28) zueinander in Abhängigkeit vom hochdruckseitigen Druckverlauf erfolgt.

6. Verfahren nach einem der vorstehenden Ansprüche, wobei das aus der Förderstrecke auslaufende Quetschelement (27, 28) relativ zu dem vorlaufenden Quetschelement (27, 28) in eine neutrale Position überführt wird.

7. Dialysemaschine mit einer Fluid von einer Niederdruckseite zu einer Hochdruckseite fördernden Peristaltikpumpe (2), wobei
die Peristaltikpumpe (2) dazu eingerichtet ist, zwischen der Niederdruckseite und der Hochdruckseite eine elastisch verformbare Fluidleitung (31) aufzunehmen, und die Peristaltikpumpe 2 eine die Fluidleitung (31) stützende Stützfläche (33) und einen Rotor (23) aufweist, und
der Rotor (23) zumindest zwei jeweils ein die Fluidleitung (31) zwischen sich und der Stützfläche (33) verformende Quetschelemente (27, 28) aufweist, wobei
die Quetschelemente (27, 28) in Rotationsrichtung relativ zueinander winkelpositionierbar ausgebildet sind,
**dadurch gekennzeichnet, dass**
die Dialysemaschine des Weiteren ein Druckmessmittel (3) zur Feststellung des einlassseitigen Drucks und/oder ein Druckmessmittel (15) zur Feststellung des ausgangsseitigen Drucks und/oder ein Druckmessmittel (3, 15) zur Feststellung des Drucks in der Fluidleitung (31) aufweist, wobei der Relativwinkel in Rotationsrichtung zwischen den Quetschelementen (27,28) auf der Grundlage des festgestellten einlassseitigen Drucks und/oder des festgestellten ausgangsseitigen Drucks und/oder des festgestellten Drucks in der Fluidleitung (31) verändert wird, insbesondere verkleinert wird.

8. Dialysemaschine nach Anspruch 7, wobei die Quetschelemente (27, 28) mit einem Kurvenstellglied, insbesondere einer Kurven- oder Nockenscheibe oder einer Kurven- oder Nockenwelle, zusammenwirken, wobei die Winkelposition der Quetschelemente (27, 28) zueinander mittels des Kurvenstellgliedes einstellbar ist.

9. Dialysemaschine nach Anspruch 7 oder 8, wobei jedes Quetschelement (27, 28) jeweils mittels einer Antriebseinheit, insbesondere mittels eines Schrittmotors (19) angetrieben ist.

10. Steuereinrichtung (17) für eine Dialysemaschine, welche eine Peristaltikpumpe (2) aufweist, die ein Fluid von einer Niederdruckseite zu einer Hochdruckseite fördert, wobei
die Peristaltikpumpe (2) dazu eingerichtet ist, zwischen der Niederdruckseite und der Hochdruckseite eine elastisch verformbare Fluidleitung (31) aufzunehmen, und die Peristaltikpumpe 2 eine die Fluidleitung (31) stützende Stützfläche (33) und einen Rotor (23) aufweist;
der Rotor (23) zumindest zwei jeweils ein die Fluidleitung (31) zwischen sich und der Stützfläche (33) verformende Quetschelemente (27, 28) aufweist;
jedes Quetschelement (27, 28) jeweils mittels einer Antriebseinheit, insbesondere mittels eines Schrittmotors (19), angetrieben ist; und
die Dialysemaschine ein Druckmessmittel (3) zur Feststellung des einlassseitigen Drucks und/oder ein Druckmessmittel (15) zur Feststellung des ausgangsseitigen Drucks und/oder ein Druckmessmittel (3, 15) zur Feststellung des Drucks in der Fluidleitung (31) aufweist,
**dadurch gekennzeichnet, dass**
die Steuereinrichtung (17) zum Bewirken einer Vorkompression zumindest eine Antriebseinheit derart steuert, dass der Relativwinkel in Rotationsrichtung zwischen den Quetschelementen (27, 28) auf der Grundlage des festgestellten einlassseitigen Drucks und/oder des festgestellten ausgangsseitigen Drucks und/oder des festgestellten Drucks in der Fluidleitung (31) verändert wird, insbesondere verkleinert wird.

## Claims

1. A method of conveying fluid in an apparatus for extracorporeal blood treatment, wherein
fluid is conveyed by means of a peristaltic pump (2) from a low-pressure side to a high-pressure side, and
an elastically deformable fluid line (31) arranged between the low-pressure side and the high-pressure side is deformed between a support surface (33) and a rotor (23) rotating vis-à-vis the latter having at least two pinch elements (27, 28), wherein
the pinch elements (27, 28) are positioned at angles relative to each other during rotation of the rotor (23) for causing pre-compression, **characterized in that**
the low-pressure side pressure and the high-pressure side pressure are sensed, a pressure difference is formed and angular positioning of the pinch elements (27, 28) relative to each other is performed depending on said angular difference.

2. The method according to claim 1, wherein between two neighboring pinch elements (27, 28) a fluid volume to be conveyed is enclosed and subsequently a volume compression of the enclosed fluid volume takes place in that the angular distance α of said pinch elements (27, 28) from each other is reduced.

3. The method according to claim 1 or 2, wherein the trailing pinch element (27, 28) is preset in the direction of the leading pinch element (27, 28).

4. The method according to any one of the preceding claims 1 to 3, wherein the high-pressure side pressure and the pressure in the conveyed fluid volume are sensed, a pressure difference is formed and the angular positioning of the pinch elements (27, 28) relative to each other is performed depending on said pressure difference.

5. The method according to any one of the preceding claims 1 to 3, wherein the high-pressure side pressure pattern is detected and the angular positioning of the pinch elements (27, 28) relative to each other is performed depending on the high-pressure side pressure pattern.

6. The method according to any one of the preceding claims, wherein the pinch element (27, 28) running out of the conveying path is transferred into a neutral position relative to the leading pinch element (27, 28).

7. A dialysis machine comprising a peristaltic pump (2) conveying fluid from a low-pressure side to a high-pressure side, wherein
the peristaltic pump (2) is arranged to receive an elastically deformable fluid line (31) between the low-pressure side and the high-pressure side and the peristaltic pump (2) includes a support surface (33) supporting the fluid line (31) and a rotor (23), and
the rotor (23) comprises at least two pinch elements (27, 28) each deforming the fluid line (31) between itself and the support surface (33), wherein
the pinch elements (27, 28) are configured to be angularly positioned relative to each other in the direction of rotation, **characterized in that**
the dialysis machine further comprises a pressure gauge (3) for determining the inlet-side pressure and/or a pressure gauge (15) for determining the outlet-side pressure and/or a pressure gauge (3, 15) for determining the pressure prevailing in the fluid line (31), wherein the relative angle in the direction of rotation is varied, especially reduced, between the pinch elements (27, 28) on the basis of the determined inlet-side pressure and/or the determined outlet-side pressure and/or the determined pressure prevailing in the fluid line (31).

8. The dialysis machine according to claim 7, wherein the pinch elements (27, 28) interact with a curve actuator, especially a curve or cam disk or a curve or cam shaft, the angular position of the pinch elements (27, 28) relative to each other being adjustable by means of the curve actuator.

9. The dialysis machine according to claim 7 or 8, wherein each pinch element (27, 28) is driven by means of a drive unit, especially by means of a step motor (19).

10. A controller (17) for a dialysis machine which includes a peristaltic pump (2) conveying fluid from a low-pressure side to a high-pressure side, wherein
the peristaltic pump (2) is arranged to receive an elastically deformable fluid line (31) between the low-pressure side and the high-pressure side and the peristaltic pump (2) comprises a support surface (33) supporting the fluid line (31) and a rotor (23);
the rotor (23) includes at least two pinch elements (27, 28) each deforming the fluid line (31) between itself and the support surface (33);
each pinch element (27, 28) is driven by means of a drive unit, in particular by means of a step motor (19); and
the dialysis machine includes a pressure gauge (3) for determining the inlet-side pressure and/or a pressure gauge (15) for determining the outlet-side pressure and/or a pressure gauge (3, 15) for determining the pressure prevailing in the fluid line (31),
**characterized in that**
the controller (17) controls at least one drive unit for causing pre-compression so that the relative angle in the direction of rotation is varied, especially reduced, between the pinch elements (27, 28) on the basis of the determined inlet-side pressure and/or the determined outlet-side pressure and/or the determined pressure prevailing in the fluid line (31).

## Revendications

1. Procédé de refoulement de fluide dans un dispositif pour le traitement sanguin extracorporel, dans lequel
du fluide est refoulé au moyen d'une pompe péristaltique (2) d'un côté basse pression à un côté haute pression, et
une conduite de fluide (31) déformable élastiquement et agencée entre le côté basse pression et le côté haute pression est déformée entre une surface d'appui (33) et un rotor (23) tournant par rapport à celle-ci avec au moins deux éléments d'écrasement (27, 28), dans lequel
les éléments d'écrasement (27, 28) sont positionnés angulairement l'un par rapport à l'autre pendant la rotation du rotor (23) pour provoquer une précompression,
**caractérisé en ce que**
une pression côté basse pression et une pression côté haute pression sont détectées, une différence de pression est formée et un positionnement angulaire des éléments d'écrasement (27, 28) l'un par rapport à l'autre est effectué en fonction de cette différence de pression.

2. Procédé selon la revendication 1, dans lequel un volume de fluide à refouler est contenu entre deux éléments d'écrasement (27, 28) contigus et ensuite une compression de volume du volume de fluide contenu est effectuée en réduisant la distance angulaire α de ces éléments d'écrasement (27, 28) l'un par rapport à l'autre.

3. Procédé selon la revendication 1 ou 2, dans lequel l'élément d'écrasement (27, 28) en aval est présenté en direction de l'élément d'écrasement (27, 28) en amont.

4. Procédé selon l'une quelconque des revendications précédentes 1 à 3, dans lequel la pression côté haute pression et la pression dans le volume de fluide refoulé sont détectées, une différence de pression est formée et le positionnement angulaire des éléments d'écrasement (27, 28) l'un par rapport à l'autre est effectué en fonction de cette différence de pression.

5. Procédé selon l'une quelconque des revendications précédentes 1 à 3, dans lequel l'évolution de pression côté haute pression est détectée et le positionnement angulaire des éléments d'écrasement (27, 28) l'un par rapport à l'autre est effectué en fonction de l'évolution de pression côté haute pression.

6. Procédé selon l'une quelconque des revendications précédentes, dans lequel l'élément d'écrasement (27, 28) en aval de l'itinéraire de refoulement est passé dans une position neutre par rapport à l'élément d'écrasement (27, 28) en amont.

7. Machine de dialyse avec une pompe péristaltique (2) refoulant du fluide d'un côté basse pression à un côté haute pression, dans laquelle
la pompe péristaltique (2) est aménagée afin de recevoir entre le côté basse pression et le côté haute pression une conduite de fluide (31) déformable élastiquement, et la pompe péristaltique 2 présente une surface d'appui (33) appuyant sur la conduite de fluide (31) et un rotor (23), et
le rotor (23) présente au moins deux éléments d'écrasement (27, 28) déformant respectivement la conduite de fluide (31) entre lui et la surface d'appui (33), dans laquelle
les éléments d'écrasement (27, 28) sont réalisés de manière positionnable angulairement dans le sens de rotation l'un par rapport à l'autre,
**caractérisée en ce que**
la machine de dialyse présente en outre un moyen de mesure de pression (3) pour la détermination de la pression côté entrée et/ou un moyen de mesure de pression (15) pour la détermination de la pression côté sortie et/ou un moyen de mesure de pression (3, 15) pour la détermination de la pression dans la conduite de fluide (31), dans laquelle l'angle relatif est modifié, en particulier est réduit, dans le sens de rotation entre les éléments d'écrasement (27, 28) sur la base de la pression côté entrée déterminée et/ou de la pression côté sortie déterminée et/ou de la pression déterminée dans la conduite de fluide (31).

8. Machine de dialyse selon la revendication 7, dans laquelle les éléments d'écrasement (27, 28) coagissent avec un organe de réglage de courbe, en particulier un disque de courbe ou de came ou un arbre à courbe ou à came, dans laquelle la position angulaire des éléments d'écrasement (27, 28) l'un par rapport à l'autre est réglable au moyen de l'organe de réglage de courbe.

9. Machine de dialyse selon la revendication 7 ou 8, dans laquelle chaque élément d'écrasement (27, 28) est entraîné respectivement au moyen d'une unité d'entraînement, en particulier au moyen d'un moteur pas à pas (19).

10. Dispositif de commande (17) pour une machine de dialyse qui présente une pompe péristaltique (2) qui refoule un fluide d'un côté basse pression à un côté haute pression, dans lequel
la pompe péristaltique (2) est aménagée afin de recevoir entre le côté basse pression et le côté haute pression une conduite de fluide (31) déformable élastiquement, et la pompe péristaltique 2 présente une surface d'appui (33) appuyant sur la conduite de fluide (31) et un rotor (23) ;
le rotor (23) présente au moins deux éléments d'écrasement (27, 28) déformant respectivement la conduite de fluide (31) entre lui et la surface d'appui (33) ;
chaque élément d'écrasement (27, 28) est entraîné respectivement au moyen d'une unité d'entraînement, en particulier au moyen d'un moteur pas à pas (19) ; et
la machine de dialyse présente un moyen de mesure de pression (3) pour la détermination de la pression côté entrée et/ou un moyen de mesure de pression (15) pour la détermination de la pression côté sortie et/ou un moyen de mesure de pression (3, 15) pour la détermination de la pression dans la conduite de fluide (31),
**caractérisé en ce que**
le dispositif de commande (17) commande au moins une unité d'entraînement pour provoquer une précompression de telle manière que l'angle relatif soit modifié, en particulier soit réduit, dans le sens de rotation entre les éléments d'écrasement (27, 28) sur la base de la pression côté entrée déterminée et/ou de la pression côté sortie déterminée et/ou de la pression déterminée dans la conduite de fluide (31).
